# EUROPEAN PATENT APPLICATION

(11) **EP 4 468 743 A1**
(43) Date of publication of application: **27.11.2024**
(21) Application number: 23743161.4
(22) Date of filing: 12.01.2023
(51) Int. Cl.: H04R 17/00, H04R 31/00, A61B 8/00, H10N 30/06, H10N 30/078, H10N 30/30, H10N 30/87, H10N 30/88

(54) **ULTRASONIC PROBE AND ULTRASONIC PROBE MANUFACTURING METHOD**

(30) Priority: 19.01.2022 JP 2022006067; 22.09.2022 JP 2022151759
(71) Applicant: Cast Inc., Kumamoto-shi, Kumamoto 862-0970 (JP); NOK Corporation, Minato-ku Tokyo 105-8585 (JP)
(72) Inventor: TANABE, Masayuki, Kumamoto-shi, Kumamoto 862-0970 (JP); TANAKA, Yuya, Kumamoto-shi, Kumamoto 862-0970 (JP); NAKATSUMA, Kei, Kumamoto-shi, Kumamoto 862-0970 (JP); SATO, Kosuke, Fujisawa-shi, Kanagawa 251-0042 (JP); FUTASHIMA, Ryo, Fujisawa-shi, Kanagawa 251-0042 (JP); ISHIKAWA, Terumitsu, Fujisawa-shi, Kanagawa 251-0042 (JP); UDA, Toru, Fujisawa-shi, Kanagawa 251-0042 (JP); TSUNAJIMA, Shunichi, Fujisawa-shi, Kanagawa 251-0042 (JP)
(74) Representative: TBK
(86) International application number: PCT/JP2023/000563
(87) International publication number: WO 2023/140166

(57) **Abstract**

An ultrasonic probe includes at least one piezoelectric element configured to transmit and receive ultrasonic waves. The at least one piezoelectric element includes a piezoelectric material formed by a sol-gel process, and at least one electrode being in contact with the piezoelectric material and containing a conductive elastomer.

## Description

### TECHNICAL FIELD

The present disclosure relates to ultrasonic probes and to a method for manufacturing ultrasonic probes.

### BACKGROUND ART

In medical diagnosis of living bodies and non-destructive testing of structural objects, ultrasonic measurement using an ultrasonic probe is utilized to test an internal structure non-invasively and non-destructively. A general ultrasonic probe has a piezoelectric element that mutually converts an electrical signal based on voltage changes to mechanical vibrations of ultrasonic waves, and vice versa. A general piezoelectric element has a piezoelectric material, an upper electrode disposed on the upper surface of the piezoelectric material, and a lower electrode disposed on the lower surface thereof.

A flexible ultrasonic probe is desired to enable accurate measurement of a curved part of an object, which means that a flexible piezoelectric element is desired to be included in the ultrasonic probe.

A piezoelectric material included in a piezoelectric element described in Patent Document 1 is formed by a sol-gel process to provide flexibility of an ultrasonic probe. An upper electrode included in the piezoelectric element is made of a conductive paste or by a metal deposition method.

### Related Art Document

### Patent Document

Patent Document 1: WO2013/063676

### SUMMARY OF THE INVENTION

### Problems to be Solved by the Invention

A piezoelectric material has large irregularities on its surface because the piezoelectric material formed by the sol-gel process is porous. In forming a metallic film on the piezoelectric material by the deposition method, it is difficult to form a sufficiently thick electrode and the surface of the piezoelectric material cannot be sufficiently covered. As a result, the conductivity of the electrode is deteriorated, resulting in unstable operations of the ultrasonic probe.

### Means for Solving the Problems

In order to solve the above problems, an ultrasonic probe according to one aspect of the present disclosure includes a piezoelectric element configured to transmit and receive ultrasonic waves. The piezoelectric element includes a piezoelectric material formed by a sol-gel process, and an electrode being in contact with the piezoelectric material and containing a conductive elastomer.

A method according to another aspect of the present disclosure is a method for manufacturing an ultrasonic probe including a piezoelectric element configured to transmit and receive ultrasonic waves. The method includes manufacturing the piezoelectric element, in which the manufacturing includes forming a piezoelectric material by a sol-gel process, and forming an electrode by applying a conductive elastomer onto the piezoelectric material and curing the conductive elastomer.

### Effect of the Invention

According to the present disclosure, it is possible to provide a flexible ultrasonic probe.

### BRIEF DESCRIPTION OF THE DRAWINGS

FIG. 1 is a plan view illustrating an ultrasonic probe according to a first embodiment.
FIG. 2 is a sectional view of the ultrasonic probe illustrated in FIG. 1.
FIG. 3 is a diagram illustrating a flow of a method for manufacturing the ultrasonic probe according to the first embodiment.
FIG. 4 is a plan view illustrating an ultrasonic probe according to a second embodiment.
FIG. 5 is a view of the ultrasonic probe of FIG. 4 as viewed in an X2 direction.
FIG. 6 is a view of the ultrasonic probe of FIG. 4 as viewed in a Y1 direction.

### MODES FOR CARRYING OUT THE INVENTION

Preferred embodiments of the present disclosure are described below with reference to the accompanying drawings. The scope of the present disclosure is not limited to the embodiments unless there are descriptions particularly limiting the present disclosure in the following explanations.

### 1. First Embodiment

### 1-1. Ultrasonic probe 100

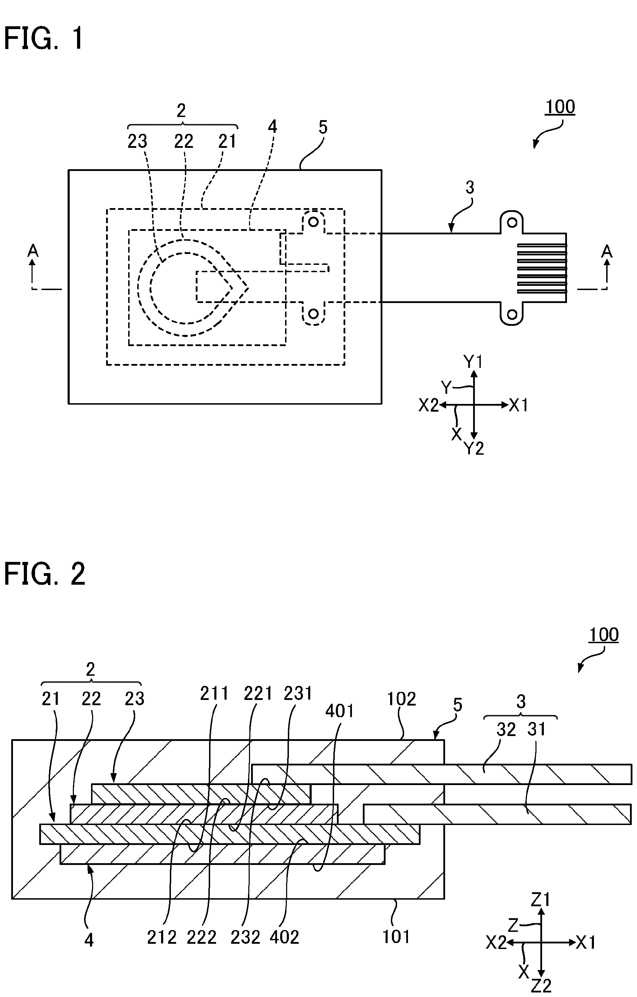

FIG. 1 is a plan view illustrating an ultrasonic probe 100 according to a first embodiment. FIG. 2 is a sectional view of the ultrasonic probe 100 illustrated in FIG. 1. The dimension and the scale of each part of the ultrasonic probe 100 illustrated in FIG. 2 are different from actual ones as appropriate and are schematically illustrated to facilitate understanding. In practice, the thickness of the ultrasonic probe 100 is considerably smaller than that illustrated in FIG. 2. FIG. 2 corresponds to a cross-sectional surface taken along line A-A in FIG. 1.

In the following descriptions, an X-axis, a Y-axis, and a Z-axis orthogonal to each other are used as required for the sake of explanations. One direction along the X-axis is represented as an X1 direction and the direction opposite to the X1 direction is represented as an X2 direction. Similarly, one direction along the Y-axis is represented as a Y1 direction and the direction opposite to the Y1 direction is represented as a Y2 direction. One direction along the Z-axis is represented as a Z1 direction, and the direction opposite to the Z1 direction is represented as a Z2 direction. In the following descriptions, viewing in the Z1 direction or the Z2 direction is referred to as "plan view." It is envisaged that the Z1 direction represents an upward direction and the Z2 direction represents a downward direction.

The ultrasonic probe 100 illustrated in FIGS. 1 and 2 are used in ultrasonic measurement, such as medical diagnosis of living bodies and non-destructive testing of structural objects. The ultrasonic measurement is a method for measuring the presence or absence of a subject or the location of the subject by transmitting an ultrasonic signal to the subject from the ultrasonic probe 100 and receiving the ultrasonic signal reflected from the subject with the ultrasonic probe 100. Ultrasonic waves have a property of reflecting on an interface between different objects. As a difference in acoustic impedance between objects is greater, the reflectance of ultrasonic waves is greater. The presence or absence of a subject or the location of the subject is measured based on the reflectance. With this ultrasonic measurement, an internal structure can be tested non-invasively and non-destructively.

The ultrasonic probe 100 is flexible. The flexible ultrasonic probe 100 makes good contact with a curved surface of the neck, the chest, the abdomen, the arms, and the fingers, for example. Thus, the ultrasonic probe 100 has good contact characteristics not only to a flat surface of a subject, but also with a curved surface. The use of the ultrasonic probe 100 can broaden the measurable ranges of a subject.

The ultrasonic probe 100 includes a piezoelectric element 2, a flexible wiring substrate 3, a backing material 4, and a housing 5. As illustrated in FIG. 2, the ultrasonic probe 100 has a lower surface 101 and an upper surface 102. The upper surface 102 comes into contact with a subject. The ultrasonic probe 100 transmits and receives ultrasonic waves from the upper surface 102.

### 1-1a. Piezoelectric element 2

The piezoelectric element 2 illustrated in FIGS. 1 and 2 transmits and receives ultrasonic waves. The piezoelectric element 2 mutually converts an electrical signal based on voltage changes to mechanical vibrations of ultrasonic waves, and vice versa.

The piezoelectric element 2 includes a lower electrode 21, a piezoelectric material 22, and an upper electrode 23. The piezoelectric material 22 is between the lower electrode 21 and the upper electrode 23 and is in contact with these electrodes. In a state in which the ultrasonic probe 100 is in contact with a subject, the lower electrode 21 is at a location farther from the subject than the upper electrode 23. The lower electrode 21 corresponds to "another electrode" that is in contact with the opposite surface of the piezoelectric material 22 to the upper electrode 23.

In an example illustrated in FIG. 1, the shape of the lower electrode 21 in plan view is rectangular. The lower electrode 21 is flexible. The material of the lower electrode 21 is, for example, a metal, such as stainless steel. The use of a metal lower electrode 21 enables the lower electrode 21 to be a thinner film than an elastomeric one, and to provide hardness of the ultrasonic probe 100 to be as low as practical. The material of the lower electrode 21 may be a metal other than stainless steel, or it may be a non-metallic material, such as carbon.

As illustrated in FIG. 2, the lower electrode 21 has a lower surface 211 and an upper surface 212. The piezoelectric material 22 is in contact with the upper surface 212. The thickness of the lower electrode 21 is, for example, preferably not less than 10 µm and not greater than 200 µm. The thickness is more preferably not less than 50 µm and not greater than 100 µm. A thickness within these ranges maintains flexibility of the ultrasonic probe 100 and practical hardness thereof in contrast to when the thickness is out of these ranges.

As illustrated in FIG. 1, the shape of the piezoelectric material 22 in plan view is substantially circular. The shape may be other than circular, such as polygonal (e.g., rectangular). In the illustrated example, the piezoelectric material 22 has its plan area smaller than the lower electrode 21. The piezoelectric material 22 overlaps with the lower electrode 21 in plan view.

The piezoelectric material 22 is a sol-gel film formed by a sol-gel process and is porous. A sol-gel process for forming the piezoelectric material 22 provides a highly flexible piezoelectric material 22. The material of the piezoelectric material 22 is piezoelectric ceramics, such as lead zirconate titanate (PZT).

As illustrated in FIG. 2, the piezoelectric material 22 has a lower surface 221 and an upper surface 222. The lower surface 221 is in contact with the lower electrode 21 and the upper surface 222 is in contact with the upper electrode 23. The thickness of the piezoelectric material 22 is preferably not less than 10 µm and not greater than 200 µm. The thickness is more preferably not less than 50 µm and not greater than 100 µm. The thickness within a such range provides easy deformation of the piezoelectric material 22 and easy formation thereof by a sol-gel process, in contrast to when the thickness is out of the range.

As illustrated in FIG. 1, the shape of the upper electrode 23 in plan view is substantially circular. The shape may be other than circular, such as polygonal (e.g., rectangular). In the illustrated example, the upper electrode 23 in plan smaller than the lower electrode 21 and the piezoelectric material 22. The upper electrode 23 overlaps with the lower electrode 21 and the piezoelectric material 22 in plan view.

The upper electrode 23 contains a conductive elastomer. Preferably, the conductive elastomer includes a binder and a conductive filler. Examples of the binder include rubber, such as silicone rubber and urethane rubber, and a thermoplastic elastomer. Examples of the conductive filler include metallic particles, such as silver powder, conductive carbon, and a plated filler, such as a silver-plated filler. The conductive filler may be powder, particles, or fibers.

The upper electrode 23 with the conductive elastomer enables provision of a thin film and provides high flexibility. This increases the capability of the upper electrode 23 to follow deformation of the piezoelectric material 22. The use of the conductive elastomer enables the upper electrode 23 having a sufficient thickness to be formed on the upper surface 222 of the piezoelectric material 22. As a result, a flexible and highly conductive ultrasonic probe 100 is provided.

Even when the upper electrode 23 is bent, the flexible upper electrode 23 suppresses the conductivity thereof from decreasing. If the upper electrode 23 is a metallic film, the flexibility of the material itself is low. When the curvature of the upper electrode 23 is small or when the upper electrode 23 is repeatedly bent, the upper electrode 23 may be damaged. However, the risk of damage (e.g., cracks) to the upper electrode 23 can be reduced even when the curvature of the upper electrode 23 is small or when the upper electrode 23 is repeatedly bent. This is because the upper electrode 23 according to this embodiment is not a metallic film and is highly flexible. As a result, decrease in the conductivity due to minute cracks on the upper electrode 23 is suppressed. The upper electrode 23 with the conductive elastomer enhances the durability of the ultrasonic probe 100.

It is preferable that silicone rubber among the forementioned conductive elastomers be included as the conductive elastomer. Silicone rubber is low in the attenuating property for ultrasonic waves. The use of silicone rubber in the upper electrode 23 provides a higher-precision ultrasonic probe 100, as compared to use of urethane rubber.

From among the conductive fillers, a silver filler is preferable as the conductive filler. Specifically, a silica core with a silica core material is an example of the silver-plated filler. Silver is highly conductive. A silver filler as the conductive filler provides the highly conductive upper electrode 23. Furthermore, the conductive filler may include two or more types of fillers differing in average particle diameter. In particular, such an upper electrode 23, which includes two or more types of silver fillers differing in average particle diameter, enhances the performance of the upper electrode 23.

The compound ratio of the conductive filler to 100 phr of the binder is preferably not less than 50 phr and not greater than 600 phr, although it is not particularly limited thereto. The compound ratio is more preferably not less than 100 phr and not greater than 400 phr. The compound ratios within these ranges provide high conductivity and easy formation of the upper electrode 23 that are less likely to be damaged, in contrast to when the compound ratio is out of these ranges.

For two or more types of conductive fillers differing in the average particle diameter, the average diameter of each of the two types is not particularly limited thereto. The average diameter of one of the two types is preferably not less than 4 µm and not greater than 8 µm. The other average diameter is preferably not less than 5 µm and not greater than 15 µm. With the average diameters equal to or greater than the lower limits, the conductive filler are less likely to aggregate, in contrast to when the average diameters are less than the lower limits. With the average diameters equal to or less than the upper limits, the upper electrode 23 is less likely to be damaged by bending with a small curvature as compared to a case in which the average diameters are greater than the upper limits.

The conductive elastomer may include a curing agent and a dispersing agent in addition to the binder and the conductive filler described above. Materials other than the forementioned materials may be included without significantly changing the characteristics of the upper electrode 23. The compound ratio of the curing agent to 100 phr of the binder is, for example, equal to or less than 10 phr. The compound ratio of the dispersing agent to 100 phr of the binder is, for example, equal to or less than 20 phr. Two or more types of each of the curing agent and the dispersing agent may be used.

As illustrated in FIG. 2, the upper electrode 23 has a lower surface 231 and an upper surface 232. The lower surface 231 is in contact with the piezoelectric material 22. The upper surface 232 is in contact with the housing 5. The thickness of the upper electrode 23 is preferably not less than 5 µm and not greater than 200 µm. The thickness is more preferably not less than 10 µm and not greater than 100 µm. The thickness within the range allows easier deformation of the upper electrode 23, suppresses attenuation of ultrasonic waves, and makes the thickness of the upper electrode 23 uniform, in contrast to when the thickness is out of the range.

As described above, the piezoelectric element 2 includes the flexible lower electrode 21, the piezoelectric material 22 formed by a sol-gel process, and the upper electrode 23 with the conductive elastomer. As a result, the piezoelectric element 2 is flexible, which ensures flexibility of the ultrasonic probe 100. The ultrasonic probe 100 can be applied not only to a flat surface of a subject but also to a curved surface, which provides wider applicable range of the ultrasonic probe 100. Furthermore, the highly flexible piezoelectric element 2 enhances the durability of the ultrasonic probe 100 because damage (e.g., cracks) is less likely to occur.

### 1-1b. Flexible wiring substrate 3

The flexible wiring substrate 3 is connected to the piezoelectric element 2. The flexible wiring substrate 3 includes a film insulating substrate, a wiring 31 disposed in the insulating substrate, and a wiring 32 disposed in the insulating substrate. In FIG. 2, the wiring 31 and the wiring 32 are schematically illustrated. The insulating substrate is made of a resin such as polyimide. The wirings 31 and 32 are made of a metal such as copper. The wiring 31 is connected to the lower electrode 21. The wiring 32 is connected to the upper electrode 23.

### 1-1c. Backing material 4

The backing material 4 is disposed under the lower electrode 21. The backing material 4 is in contact with the lower surface 211 of the lower electrode 21, which is the opposite surface to the piezoelectric material 22. The backing material 4 is flexible. The backing material 4 attenuates ultrasonic waves emitted downward from the piezoelectric element 2 and suppresses undesired vibrations. The provision of the backing material 4 suppresses undesired vibrations and enhances the resolution in contrast when no backing material 4 is provided. In addition, the provision of the backing material 4 reduces multiple reflections between the lower surface 101 of the ultrasonic probe 100 and the lower surface 211 of the piezoelectric element 2.

The shape of the backing material 4 in plan view is rectangular in the illustrated example. The shape of the backing material 4 in plan view may be other than rectangular, for example, circular. The backing material 4 has its plan area greater than the piezoelectric material 22 and overlaps with the piezoelectric material 22 in plan view. Such a backing material 4, which has its plan area greater than the piezoelectric material 22, more effectively achieves the function to attenuate ultrasonic waves emitted downward from the piezoelectric material 22, in contrast to a backing material 4 with its small plan area.

It is preferable that the material of the backing material 4 be an elastomer. The use of an elastomer as the material provides a flexible backing material 4. Specifically, the material of the backing material 4 is preferably butyl rubber. Butyl rubber has a greater attenuating property for ultrasonic waves than, for example, silicone rubber. The use of butyl rubber as the material of the backing material 4 attenuates ultrasonic waves sufficiently even when the thickness is small. It is preferable that the backing material 4 contain no inorganic particles, which increases the flexibility of the backing material 4 in contrast to use of the inorganic particles.

As illustrated in FIG. 2, the backing material 4 has a lower surface 401 and an upper surface 402. The lower surface 401 is in contact with the housing 5. The upper surface 402 is in contact with the piezoelectric element 2. The thickness of the backing material 4 is preferably not less than 0.1 mm and not greater than 1.0 mm. The thickness is more preferably not less than 0.2 mm and not greater than 0.8 mm. The thickness within this range provides a highly flexible backing material 4, and in particular, is more effective in attenuating ultrasonic waves, in contrast to when the thickness is outside of this range.

### 1-1d. Housing 5

The housing 5 illustrated in FIG. 1 covers the piezoelectric element 2 and the backing material 4. A part of the flexible wiring substrate 3 is positioned in the housing 5 and the remaining part is exposed from the housing 5. The housing 5 is flexible. The housing 5 protects the piezoelectric element 2 and other elements.

It is preferable that the material of the housing 5 be an elastomer. The use of an elastomer as the material for the housing 5 provides a high flexibility, a shock absorbing property, and a water-proof property to protect the piezoelectric element 2. It is preferable that an elastomer low in the ultrasonic-wave attenuating property be used as the material of the housing 5, which protects the piezoelectric element 2 and other elements, and acts as an acoustic matching layer. Such a housing 5, which contains an elastomer low in the ultrasonic-wave attenuating property, protects the piezoelectric element 2 and other elements, and acts as an acoustic matching layer.

The acoustic matching layer is used to reduce reflection of ultrasonic waves resulting from a difference in acoustic impedance between the piezoelectric element 2 and a subject. For a living body, the acoustic impedance greatly differs between a piezoelectric element 2 and a living body. The provision of the housing 5, which acts as the acoustic matching layer, reduces reflection of ultrasonic waves resulting from the difference in the acoustic impedance between the piezoelectric element 2 and a living body. The housing 5, which acts as the acoustic matching layer, improves matching of the acoustic impedance and increases the amount of ultrasonic waves transmitted to the subject.

The material of the housing 5 is preferably silicone rubber. Silicone rubber is lower in ultrasonic-wave attenuating property than urethane rubber and other similar rubbers. A housing 5 with silicone rubber is suitably used as the acoustic matching layer and acts as an acoustic lens with an appropriate shape. An acoustic lens is used to focus ultrasonic waves to increase resolution. As a result, the provision of the housing 5, which acts as an acoustic lens, enhances resolution, in contrast to when a housing 5 is not provided. Furthermore, silicone rubber is highly biocompatible and is easily use in medical diagnoses of living bodies. Silicone rubber has an acoustic impedance that is similar to that of living bodies. For a living body as the subject, the use of silicone rubber increases the amount of ultrasonic waves transmitted to the subject and enhances resolution. For the upper electrode 23 with silicone rubber, the housing 5 with silicone rubber also enhances contact therebetween.

It is preferable that inorganic particles not be contained in the housing 5. If inorganic particles are not used, this increases the flexibility of the housing 5, in contrast to when inorganic particles are used. The use of elastomers without inorganic particles as materials of the housing 5 and the backing material 4 prevents the ultrasonic probe 100 from degrading flexibility. As a result, contact with parts of a living body is particularly increased, and a wider range of a living body can be diagnosed.

The shape of the housing 5 in plan view is rectangular in the illustrated example. The shape of the housing 5 in plan view may be other than rectangular (e.g., circular). The plan area of the housing 5, that is, the plan area of the ultrasonic probe 100 is not less than 50 mm² and not greater than 10000 mm², although this is not particularly limited thereto.

As illustrated in FIG. 2, the lower surface 101 of the ultrasonic probe 100 corresponds to the lower surface of the housing 5. The upper surface 102 corresponds to the upper surface of the housing 5. The thickness of the ultrasonic probe 100 is preferably not less than 0.1 mm and not greater than 10 mm. The thickness is more preferably not less than 0.5 mm and not greater than 2 mm. The thickness within this range provides an ultrasonic probe 100 that is highly flexible and is easier to handle, in contrast to when the thickness is outside this range.

### 1-2. Method for manufacturing the ultrasonic probe 100

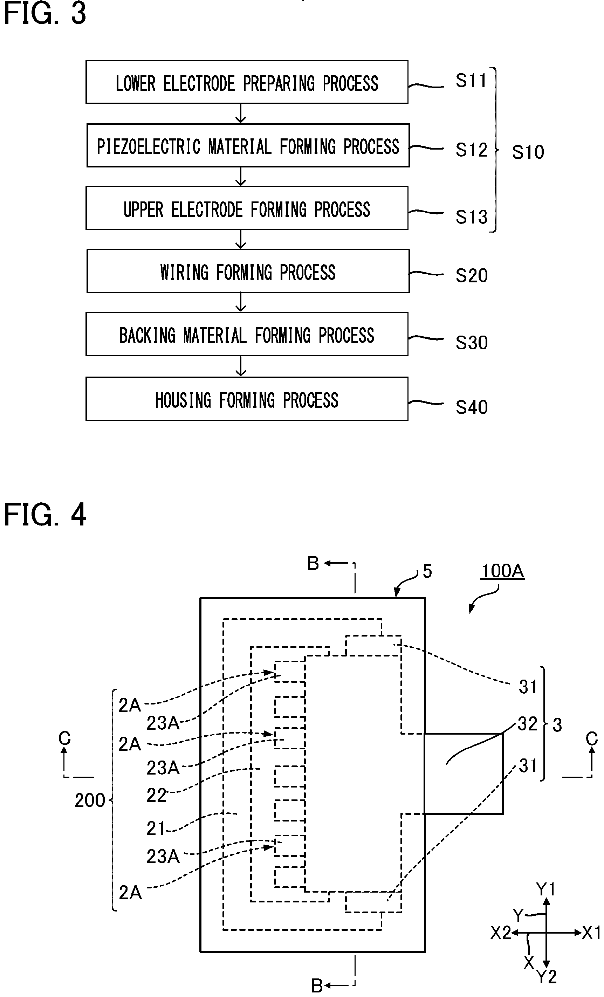

FIG. 3 is a diagram illustrating a flow of a method for manufacturing the ultrasonic probe 100 according to the first embodiment. As illustrated in FIG. 3, the method for manufacturing the ultrasonic probe 100 includes a piezoelectric element forming process S10, a wiring forming process S20, a backing material forming process S30, and a housing forming process S40. The piezoelectric element forming process S10 includes a lower electrode preparing process S11, a piezoelectric material forming process S12, and an upper electrode forming process S13.

At the piezoelectric element forming process S10, the piezoelectric element 2 is formed. Specifically, first, at the lower electrode preparing process S11, the lower electrode 21 is prepared. For example, a stainless steel substrate with a thickness of 50 µm is prepared.

At the piezoelectric material forming process S12, the piezoelectric material 22 is formed on the lower electrode 21 by a sol-gel process. With the formation of the piezoelectric material 22 by a sol-gel process, a flexible and thin piezoelectric material 22 can be easily formed. Specifically, a sol-gel film with a thickness of about 100 µm is formed on the lower electrode 21.

At the upper electrode forming process S13, the upper electrode 23 is formed, for example, by applying a conductive elastomer onto the piezoelectric material 22 by a screen method and curing the conductive elastomer. Specifically, first, the following are combined to form a conductive elastomer: (i) 100 phr of a binder "KE-106" manufactured by Shin-Etsu Chemical Co., Ltd., (ii) 150 phr of silver filler "FA-2-3" manufactured by DOWA ELECTRONICS MATERIALS CO., Ltd., (iii) 150 phr of silver filler "G-35" manufactured by DOWA ELECTRONICS MATERIALS CO., Ltd., (iv) 10 phr of a curing agent "CAT-RG" manufactured by Shin-Etsu Chemical Co., Ltd., (v) 10 phr of "KF-6015" manufactured by Shin-Etsu Chemical Co., Ltd., and (vi) 10 phr of "KF-6106" manufactured by Shin-Etsu Chemical Co., Ltd. Next, the conductive elastomer is applied onto the piezoelectric material 22 with a thickness not less than 50 µm and not greater than 100 µm and is cured at 150 °C for 30 minutes. If the conductive elastomer is applied in a substantially circular manner in plan view, the diameter thereof is about not less than 6 mm and not greater than 9 mm.

Using the conductive elastomer, the conductive elastomer can be applied onto the piezoelectric material 22 without the material of the upper electrode 23 permeating into the piezoelectric material 22. Furthermore, using the conductive elastomer, the upper electrode 23 with a sufficient thickness can be formed on the piezoelectric material 22, which leads to the ultrasonic probe 100 with a satisfactory conductivity.

The conductive elastomer contains a binder and a conductive filler. The binder is a rubber, such as silicone rubber or urethane rubber or a thermoplastic elastomer. Curing of the binder prevents movement of the conductive filler. The use of the conductive elastomer makes it possible to form the upper electrode 23, in which damage (e.g., cracks) is less likely to be produced even when the upper electrode 23 is deformed, and in which decrease in conductivity is suppressed.

The piezoelectric element 2 is formed by the foregoing processes. As described above, the piezoelectric element 2 includes the piezoelectric material 22 formed by a sol-gel process, and the upper electrode 23 made of the conductive elastomer. As a result, the piezoelectric element 2 is flexible. The flexible piezoelectric element 2 ensures a flexible ultrasonic probe 100. This enables the ultrasonic probe 100 to be applied not only to a flat surface of a subject but also to a curved surface. The applicable range of the ultrasonic probe 100 can be broadened. Furthermore, since the ultrasonic probe 100 is highly flexible, damage, such as cracks, are less likely to occur, and the durability of the ultrasonic probe 100 is improved.

At the wiring forming process S20, the flexible wiring substrate 3 connected to the piezoelectric element 2 is formed. Specifically, the flexible wiring substrate 3 has a part in which a conductive member, such as copper, is exposed. In this case, first, an anisotropic conductive film, which acts as an adhesive member, is temporarily pressure-joined to this part. More specifically, an anisotropic conductive film "MF-331" manufactured by Hitachi Chemical Company, Ltd., is temporarily pressure-joined to a 1.5-millimeter-square exposed part of copper at 80 °C for 3 seconds.

Subsequently, the wiring 32 of the flexible wiring substrate 3 and the upper electrode 23 are thermocompression-bonded to each other. For the upper electrode 23 made of silicone rubber, it is preferable that the thermocompression bonding be performed after ultraviolet light is emitted on the surface of the upper electrode 23 to reform the surface. Specifically, vacuum ultraviolet light is applied for 30 seconds using an excimer lamp to reform the surface of the upper electrode 23. Subsequently, the wiring 32 and the upper electrode 23 are thermocompression-bonded to each other at 140 °C for 10 seconds using an FPC alignment thermocompression bonding device. The wiring 31 of the flexible wiring substrate 3 and the lower electrode 21 are also thermocompression-bonded to each other. Specifically, the wiring 31 and the lower electrode 21 are thermocompression-bonded to each other at 140 °C for 10 seconds using the FPC alignment thermocompression bonding device.

In the backing material forming process S30, the backing material 4 is formed. Specifically, the backing material 4 is bonded to the lower surface 211 of the lower electrode 21 by applying pressure to form the backing material 4 on the lower surface 211. If the backing material 4 contains butyl rubber and the housing 5 contains silicone rubber, it is preferable that an adhesive butyl rubber tape be used as the backing material 4. Specifically, a butyl rubber tape "No. 5938" manufactured by Maxell Sliontec Ltd., is bonded as the backing material 4.

At the housing forming process S40, the housing 5 is formed. For example, the housing 5 is formed by sticking a sheet that contains a self-adhesive elastomer with an adhesive to the upper surface 232 of the upper electrode 23 and the lower surface 401 of the backing material 4. Specifically, a self-adhesive silicone rubber sheet is stuck to each of the upper surface 232 and the lower surface 401 with an adhesive. Given the amount of ultrasonic waves transmitted to a subject, the thickness of the sheet that is in contact with the upper surface 232 of the upper electrode 23 is less than that of the thickness of the sheet that is in contact with the lower surface 401 of the backing material 4. For example, a self-adhesive silicone rubber sheet with a thickness of 0.4 mm is stuck onto the upper surface 232. Another self-adhesive silicone rubber sheet with a thickness of 0.6 mm is stuck onto the lower surface 401.

For example, if the housing 5 is made of sheets with silicone rubber, it is preferable that these sheets be stuck to each other after applying ultraviolet light to the surfaces of the sheets to reform the surfaces because silicone rubber is poor in adhesive property. Specifically, the surfaces of the sheets are reformed by applying vacuum ultraviolet light for 30 seconds using an excimer lamp.

The adhesion temperature of the sheet on the upper surface 232 and the sheet of the lower surface 401 is an ordinary temperature, the pressure is 1.2 MPa, and the adhesion time is 30 minutes. At the time of sticking of the sheets to each other, pressurization may be performed through a foam sheet. For example, after the sheets are stuck to each other, the sheets are left for about 24 hours to obtain a practical strength.

The ultrasonic probe 100 is manufactured in the forementioned manner. By the foregoing method, a flexible ultrasonic probe 100 with a satisfactory resolution is easily manufactured.

### 2. Second Embodiment

A second embodiment of the present disclosure is explained below. In the embodiment exemplified below, elements having operations and functions identical to those of the first embodiment are denoted by signs used in the explanations of the first embodiment and detailed explanations of such elements are omitted as appropriate.

### 2-1. Ultrasonic probe 100A

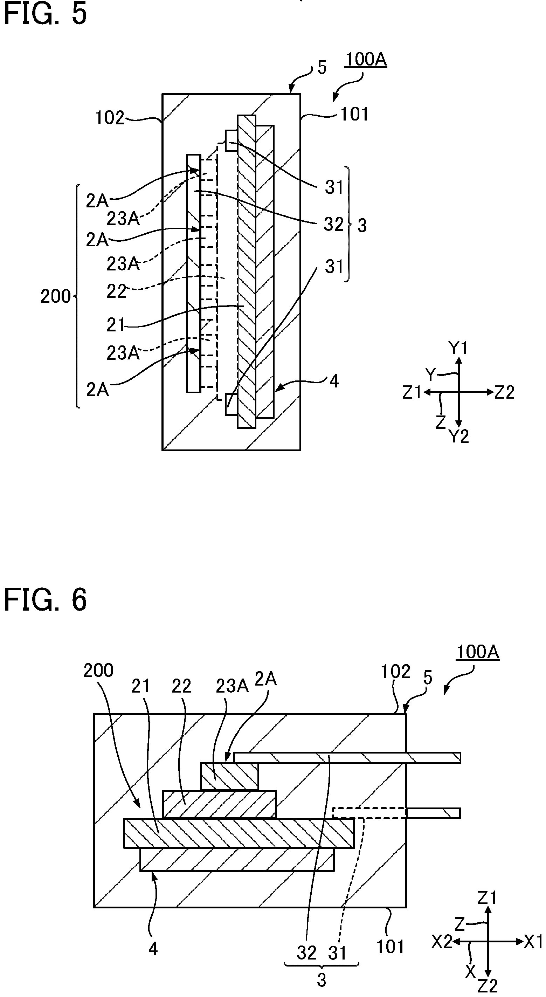

FIG. 4 is a plan view illustrating an ultrasonic probe 100A according to the second embodiment. FIG. 5 is a view of the ultrasonic probe 100A of FIG. 4 as viewed in the X2 direction. FIG. 6 is a view of the ultrasonic probe 100A of FIG. 4 as viewed in the Y1 direction. The dimensions and the scales of each part of the ultrasonic probe 100A illustrated in FIGS. 4, 5, and 6 are different from actual ones as appropriate and are schematically illustrated to facilitate understanding. FIG. 5 corresponds to a cross-sectional surface taken along line B-B in FIG. 4. FIG. 6 corresponds to a cross-sectional surface taken along line C-C in FIG. 4.

As illustrated in FIG. 4, 5, or 6, the ultrasonic probe 100A of the second embodiment is different from the ultrasonic probe 100 of the first embodiment in having a plurality of piezoelectric elements 2A.

The ultrasonic probe 100A includes a piezoelectric structure 200, the flexible wiring substrate 3, the backing material 4, and the housing 5. The piezoelectric structure 200 includes the piezoelectric elements 2A. In an example illustrated in FIG. 4, the ultrasonic probe 100A includes seven piezoelectric elements 2A. However, the number of the piezoelectric elements 2A is not limited thereto. The number may be not less than two and not more than six, or it may be equal to or more than eight. Specifically, the ultrasonic probe 100A may include piezoelectric elements 2A, such as 128 piezoelectric elements 2A.

As illustrated in FIG. 4, 5, or 6, the piezoelectric structure 200 includes the lower electrode 21, the piezoelectric material 22, and upper electrodes 23A. The lower electrode 21 is provided as one unit in common with the piezoelectric elements 2A. The piezoelectric material 22 is provided as one unit in common with the piezoelectric elements 2A.

The upper electrodes 23A correspond to "electrodes." The upper electrodes 23A are provided one-to-one for the piezoelectric elements 2A. The shape of each of the upper electrodes 23A in plan view is rectangular. The shape may be that of a polygon other than a rectangle, or it may be circular. The upper electrodes 23A are arranged in a line along the Y-axis spaced apart from each other in plan view. The center distance, that is, the pitch of the upper electrodes 23A is, for example, about 0.5 mm although it is not particularly limited thereto.

It is unnecessary that the upper electrodes 23A be arranged in a line as illustrated in FIG. 4. For example, the upper electrodes 23A may be arranged in lines or may be arranged in a random manner.

The wiring 31 is connected to the lower electrode 21 and the wiring 32 is connected to the upper electrodes 23.

The present embodiment includes piezoelectric elements 2A. The upper electrodes 23A are provided one-to-one for the piezoelectric elements 2. The piezoelectric material 22 is provided as one unit in common with the piezoelectric elements 2A. As a result, only a portion of the piezoelectric material 22 to which a voltage is applied is vibrated. In particular, the piezoelectric material 22 is formed by a sol-gel process, and it is porous and thin. Only a portion of the piezoelectric material 22 to which a voltage is applied is adequately vibrated, as long as the upper electrodes 23A are provided for the respective piezoelectric elements 2A without provision of the piezoelectric materials 22 for the respective piezoelectric elements 2A. This results in utilizing the piezoelectric material 22 for each upper electrode 23A, even when the piezoelectric material 22 is not divided. Furthermore, the piezoelectric material 22 is flexible because it is formed by the sol-gel process, and the piezoelectric material 22 is provided as one unit in common with the piezoelectric elements 2A. Therefore, the piezoelectric material 22 has a satisfactory capacity to follow bending of a subject with a curved surface.

For example, to display images of the ultrasonic probe 100 for medical use, the piezoelectric elements 2A are required. By providing the piezoelectric elements 2A, the ultrasonic probe 100 can be used as a phased array probe.

### 2-2. Method for manufacturing the ultrasonic probe 100A

In the present embodiment, a method for manufacturing the ultrasonic probe 100A includes the processes illustrated in FIG. 3 similarly to the first embodiment.

First, at the lower electrode preparing process S11, the lower electrode 21 is prepared. Specifically, a stainless steel substrate is prepared with a plan area of 25 mm × 84 mm and a thickness of 50 µm.

At the piezoelectric material forming process S12, the piezoelectric material 22 is formed on the lower electrode 21 by a sol-gel process. Specifically, a sol-gel film with a plan area of 15 mm × 80 mm and a thickness of about 100 µm is formed on the lower electrode 21. This sol-gel film is the piezoelectric material 22.

At the upper electrode forming process S13, the upper electrodes 23 are formed, for example, by applying a conductive elastomer onto the piezoelectric material 22 by a screen method and curing the conductive elastomer. For example, a conductive elastomer is applied onto the piezoelectric material 22 and is cured at 150 °C for 30 minutes. Specifically, a conductive elastomer is applied in a rectangular pattern having a plan area of 4.5 mm × 0.25 mm and a thickness of about 50 µm. For example, 128 rectangular patterns are formed. The 128 rectangular patterns are formed with a pitch of 0.5 mm, for example.

In the present embodiment, similarly to the first embodiment, the flexible ultrasonic probe 100A with a satisfactory resolution can be easily manufactured.

The present disclosure has been described based on the preferred embodiments, but it is not limited to the foregoing embodiments. The configuration of each part in the present disclosure may be replaced with any configuration that provides functions substantially identical to those of the embodiments, or any configuration may be added thereto.

In the foregoing embodiments, there is one piezoelectric element 2, but there may be more than one piezoelectric element 2. That is, the ultrasonic probe 100 may be an array probe.

### Description of Reference Signs

2... piezoelectric element, 3... flexible wiring substrate, 4... backing material, 5... housing, 21... lower electrode, 22... piezoelectric material, 23... upper electrode, 31... wiring, 32... wiring, 100... ultrasonic probe, 101... lower surface, 102... upper surface, 211... lower surface, 212... upper surface, 221... lower surface, 222... upper surface, 231... lower surface, 232... upper surface, 401... lower surface, 402... upper surface.

## Claims

1. An ultrasonic probe comprising:
at least one piezoelectric element configured to transmit and receive ultrasonic waves,
wherein the at least one piezoelectric element comprises:
a piezoelectric material formed by a sol-gel process, and
at least one electrode being in contact with the piezoelectric material and containing a conductive elastomer.

2. The ultrasonic probe according to claim 1, wherein the conductive elastomer contains silicone rubber.

3. The ultrasonic probe according to claim 1 or 2, wherein the conductive elastomer contains a conductive filler.

4. The ultrasonic probe according to claim 3, wherein the conductive filler is a silver filler.

5. The ultrasonic probe according to claim 1, further comprising:
another electrode being in contact with a surface of the piezoelectric material opposite to the at least one electrode; and
a backing material being in contact with a surface of the another electrode opposite to the piezoelectric material,
wherein the backing material contains an elastomer.

6. The ultrasonic probe according to claim 5, wherein the backing material contains butyl rubber.

7. The ultrasonic probe according to claim 5 or 6, wherein the backing material contains no inorganic particles.

8. The ultrasonic probe according to claim 1, further comprising: a housing configured to protect the piezoelectric element,
wherein the housing contains an elastomer.

9. The ultrasonic probe according to claim 8, wherein the housing contains silicone rubber.

10. The ultrasonic probe according to claim 8 or 9, wherein the housing contains no inorganic particles.

11. The ultrasonic probe according to claim 1, wherein the piezoelectric material is porous.

12. The ultrasonic probe according to claim 1, wherein:
the at least one piezoelectric element comprises a plurality of piezoelectric elements,
the at least one electrode comprises a plurality of electrodes,
the plurality of electrodes are provided corresponding to one-to-one for the plurality of piezoelectric elements, and
the piezoelectric material is provided as one unit for the plurality of piezoelectric elements.

13. A method for manufacturing an ultrasonic probe including at least one piezoelectric element configured to transmit and receive ultrasonic waves, the method comprising:
manufacturing the at least one piezoelectric element,
wherein the manufacturing comprises:
forming a piezoelectric material by a sol-gel process; and
forming at least one electrode by applying a conductive elastomer onto the piezoelectric material and curing the conductive elastomer.
